Europäisches Patentamt

European Patent Office

Office européen des brevets

(11) Veröffentlichungsnummer: **0 149 975**
**A1**

## (12) EUROPÄISCHE PATENTANMELDUNG

(21) Anmeldenummer: **84810617.5**

(22) Anmeldetag: **14.12.84**

(51) Int. Cl.⁴: **A 61 F 2/34**

(30) Priorität: **11.01.84 CH 121/84**

(43) Veröffentlichungstag der Anmeldung:
**31.07.85 Patentblatt 85/31**

(84) Benannte Vertragsstaaten:
**AT BE DE FR GB**

(71) Anmelder: **Robert Mathys Co**
**Güterstrasse 5**
**CH-2544 Bettlach(CH)**

(72) Erfinder: **Mathys, Robert, Dr. med. h.c.**
**Bahnhofstrasse 10**
**CH-2544 Bettlach(CH)**

(74) Vertreter: **Eder, Carl E. et al,**
**Patentanwaltsbüro Eder & Cie Münchensteinerstrasse 2**
**CH-4052 Basel(CH)**

(54) Hüftgelenkpfanne.

(57) Es handelt sich um eine künstliche Hüftgelenkpfanne (1) aus einem Kunststoff, die dazu bestimmt ist, ohne Knochenzement in das Becken (14) eingesetzt zu werden. Sie weist eine gerillte Aussenfläche (3) auf, die das Einwachsen des Knochens ermöglicht, sowie zwei aus der Aussenfläche (3) herausragende Sicherungszapfen (10, 11), die dazu bestimmt sind, in entsprechende Bohrungen im Knochen (14) eingesetzt zu werden. Dabei soll der Winkel, den die Zapfen (10, 11) mit der Pfannenoberfläche bilden, vom Winkel, den die Bohrungen mit der Oberfläche bilden ca. 5° abweichen, um eine gewisse, die Pfanne an ihrem Platz sichernde Vorspannung zu bewirken. Des weitern weist die Pfanne auf ihrer Aussenfläche (3) mindestens zwei widerhakenartige Verankerungslappen (12, 13) auf, die einander gegenüber liegen oder auch einen Winkelabstand von 120° - 150° voneinander aufweisen können. Durch diese Lappen wird auch dann direkt nach dem Einsetzen der Pfanne in den Knochen ein guter Sitz gewährleistet, wenn die Sicherungszapfen (10, 11) aus irgendwelchen Gründen nicht genügend Halt bieten, so dass auf die Verwendung von Schrauben oder Dübeln ganz oder teilweise verzichtet werden kann.

Fig. 4

EP 0 149 975 A1

Robert Mathys Co., Bettlach (Schweiz)

Hüftgelenkpfanne

_____

Die vorliegende Erfindung betrifft eine Weiterentwicklung und eine Verbesserung der zum zementfreien Einsetzen bestimmten Hüftgelenkpfanne nach der schweizerischen Patentschrift 544 544, also eine aus Kunststoff bestehende, künstliche Hüftgelenkpfanne mit zwei, vorzugsweise parallelen, aus der Pfannenaussenseite herausragenden Sicherungszapfen.

Die neue Pfanne ist dadurch gekennzeichnet, dass auf der Pfannenaussenseite zusätzlich mindestens zwei widerhakenartige Verankerungslappen ausgeformt sind.

Nachfolgend wird anhand der beiliegenden Zeichnung ein Ausführungsbeispiel der Erfindung beschrieben. In der Zeichnung zeigt

die Figur 1    eine Seitenansicht einer erfindungsgemässen Gelenkpfanne

die Figur 2    eine Untenansicht der gleichen Pfanne,

die Figur 3    einen Schnitt nach der Linie III - III der Fig. 2,

16541/Ed/sr/Fall 13

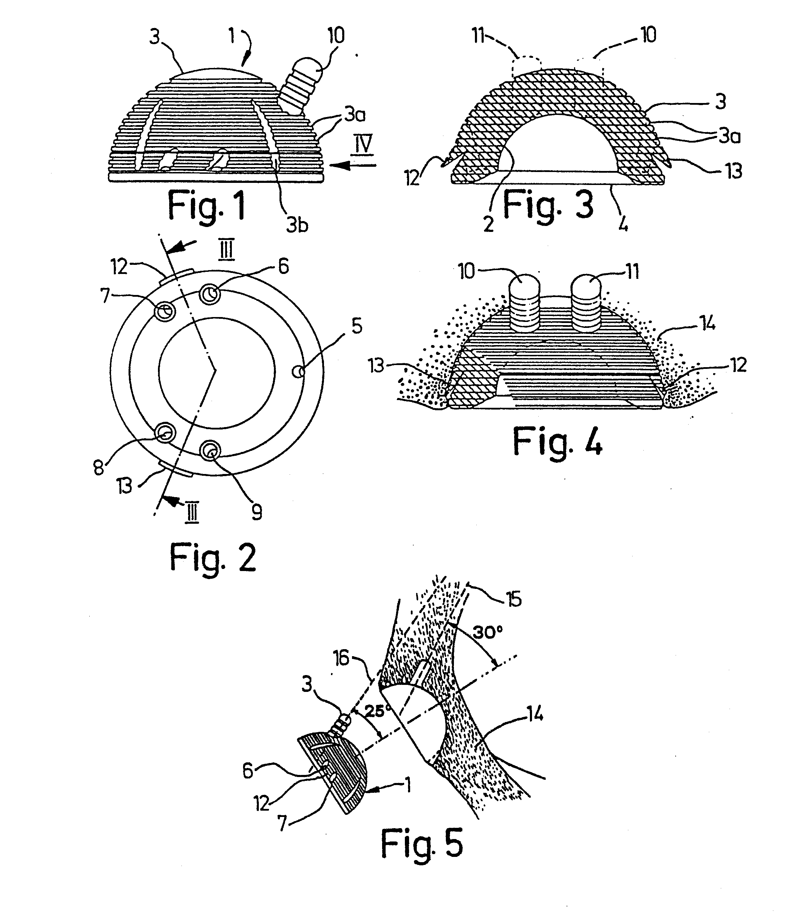

die Figur 4      eine in einem Knochen eingewachsene
                 Gelenkpfanne, teilweise in der Seitenansicht in Richtung des Pfeiles IV in
                 der Fig. 3, teilweise im Schnitt nach
                 der Linie III - III der Fig. 2 und

die Figur 5      die gleiche Pfanne vor dem Einsetzen in
                 den vorbereiteten Knochen.

Die in der Zeichnung dargestellte und als Ganzes mit 1 bezeichnete Hüftgelenkpfanne besteht aus einem gewebe- und knochenverträglichen Kunststoff, wie z.B. einem Polyäthylen, der sich für eine derartige Verwendung bewährt hat. Diese Pfanne weist die Form einer halben Hohlkugel auf, wobei die Innen-Fläche 2 glatt poliert ist, während die Aussenfläche 3 mit zur Öffnungsebene 4 parallelen Rillen 3a und einigen senkrecht dazu verlaufenden Rinnen 3b versehen ist, die alle dazu dienen, dass das Knochenmaterial in sie hineinwachsen kann, um so einen festen Verbund zwischen der künstlichen Gelenkpfanne und dem Knochen zu gewährleisten. In einer der Rillen 3a sitzt ein Metallring, der dazu dient, dass auf dem Röntgenbild die Position der Pfanne festgestellt werden kann. Vier Bohrungen 6, 7, 8 und 9, die zur Öff- nungsebene leicht schief stehen, dienen dazu, dass der Chirurg die Pfanne mittels Schrauben oder Dübeln im Becken für eine kürzere Zeit oder auf für dauernd fixieren kann, wenn er dies aus irgendwelchen Gründen für nötig hält. Eine weitere Bohrung 5 dient dazu, dass die Pfanne mittels eines Instruments gut gehalten werden kann. Auf der Aussenseite der Pfanne sind des weitern zwei Sicherungzapfen 10 und 11 angeordnet, die zueinander einigermassen parallel verlaufen, jedoch etwas schief auf der Pfannenaussenfläche 3 stehen. Diese Sicherungszapfen sind elastisch, so dass sie sich auch dann in entsprechende Bohrlöcher einschieben lassen, wenn die Achsen 15 dieser Bohrungen mit den Zapfenachsen 16 einen Winkel von 5° bilden, wie das in der Figur 5 dargestellt ist. In einem solchen Fall dienen diese Stifte 10 und 11 dank ihrer Vorspannung dazu, die Gelenkpfanne 1 an der eingesetzten Stelle mechanisch zu verankern d.h. sowohl gegen eine Drehbewegung wie auch gegen ein Herausfallen zu sichern.

Je nach den lokalen Verhältnissen lässt sich nun nicht bei allen Patienten durch diese Zapfen eine optimale Verankerung erreichen. Daher weist die erfindungsgemässe Hüftgelenkprothese auf ihrer Aussenseite 3 zusätzlich noch mindestens zwei widerhakenartige Verankerungslappen 12 und 13 auf, die, wie man aus der Fig. 4 ersehen kann, im Knochen 14, in welchem die Pfanne eingesetzt ist, eine Verankerung bewirken und die Pfanne gegen Bewegung auch dann sichern, wenn die Zapfen 10 und 11 aus irgendwelchen Gründen nicht genügende Sicherungsfunktion übernehmen können, so dass der Chirurg im allgemeinen davon absehen kann, für die Sicherung vor dem An- und Einwachsen, Schrauben oder Dübeln zu verwenden.

Für das Einsetzen einer solchen Hüftgelenkprothese gelten im Prinzip sowohl für die Indikation als auch für das Einsetzen die gleichen Regeln wie für die bekannte Hüftgelenkpfanne, mit der Ausnahme natürlich, dass es infolge der Verankerungslappen möglich ist, auf sonst allenfalls nötige Schrauben oder Dübeln zu verzichten.

## PATENTANSPRÜCHE

1. Aus Kunststoff bestehende, zum zementfreien Einsetzen bestimmte Hüftgelenkpfanne (1) mit zwei aus der Pfannenaussenseite (3) herausragenden Sicherungszapfen dadurch gekennzeichnet, dass auf der Pfannenaussenseite zusätzlich mindestens zwei widerhakenartige Verankerungslappen (12, 13) ausgeformt sind.

2. Hüftgelenkpfanne nach Anspruch 1, dadurch gekennzeichnet, dass die beiden Verankerungslappen (12, 13) einen auf dem Umfang gemessenen Bogenabstand von 120° - 150° aufweisen, wobei die Sicherungszapfen (10, 11) auf der diesem Bogenabstand gegenüberliegenden Seite angeordnet sind.

3. Hüftgelenkpfanne nach Anspruch 1 oder 2, dadurch gekennzeichnet, dass die beiden Verankerungslappen (12, 13) in der Nähe des Pfannenrandes angeordnet sind.

0149975

Fig. 1

Fig. 2

Fig. 3

Fig. 4

Fig. 5

**0149975**

Nummer der Anmeldung

## EUROPÄISCHER RECHERCHENBERICHT

EP 84 81 0617

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| D,Y | CH-A- 544 544 (MATHYS) <br> * Figuren 1, 2 * | 1 | A 61 F 2/34 |
| Y | DE-A-2 857 297 (ORTHOPLANT) <br> * Anspruch 1; Figuren 1-3 * | 1 | |
| A | EP-A-0 038 903 (GEBR. SULZER AG.) | | |
| A | DE-A-2 911 754 (REIMER) | | |
| P,X | DE-A-3 228 113 (SCHÜTT UND GRUNDEI GMBH) <br> * Ansprüche 1, 2; Figuren 1-3 * | 1-3 | |

| | | | RECHERCHIERTE SACHGEBIETE (Int. Cl.4) |
|---|---|---|---|
| | | | A 61 F 2/00 |

Der vorliegende Recherchenbericht wurde für alle Patentansprüche erstellt.

| Recherchenort <br> BERLIN | Abschlußdatum der Recherche <br> 13-03-1985 | Prüfer <br> KANAL P K |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82